# EUROPEAN PATENT APPLICATION

(11) **EP 4 230 729 A1**
(43) Date of publication of application: **23.08.2023**
(21) Application number: 21880541.4
(22) Date of filing: 14.10.2021
(51) Int. Cl.: C12N 5/0783, C12N 5/00, A61K 35/17, A61K 48/00, A61P 35/00

(54) **NATURAL KILLER CELL HAVING CONTROLLED ONCOLOGY-RELEATED GENE EXPRESSION, AND USE THEREOF**

(30) Priority: 16.10.2020 KR 20200134623
(71) Applicant: Sungkwang Medical Foundation, Seoul 06135 (KR); Cha Biolab Co., Ltd., Seongnam-si, Gyeonggi-do 13488 (KR)
(72) Inventor: BAEK, Young Seok, Seongnam-si, Gyeonggi-do 13488 (KR); AN, Hee Jung, Seoul 06098 (KR); KANG, Myung Seo, Seoul 06714 (KR); MOON, Yong Wha, Seongnam-si, Gyeonggi-do 13568 (KR); PARK, Kyung Soon, Seongnam-si, Gyeonggi-do 13565 (KR); LEE, In Jee, Seongnam-si, Gyeonggi-do 13488 (KR); HWANG, So Hyun, Seoul 05823 (KR)
(74) Representative: Mewburn Ellis LLP
(86) International application number: PCT/KR2021/014245
(87) International publication number: WO 2022/080894

(57) **Abstract**

Provided are a method of culturing high-purity, highly active natural killer cells of which expression of anti-cancer-related gene is regulated, and use thereof for anticancer immunotherapy. In the natural killer cells according to one aspect, the expression of anticancer related genes is significantly increased or decreased before and after culture. The natural killer cells have the unique characteristics thereof, and remarkably high activity and proliferation fold. Therefore, the natural killer cells can be used for effective anticancer immunotherapy.

## Description

### CROSS-REFERENCE TO RELATED APPLICATION

This application claims the benefit of Korean Patent Application No. 10-2020-0134623, filed on October 16, 2020, in the Korean Intellectual Property Office, the disclosure of which is incorporated herein in its entirety by reference.

### BACKGROUND

### 1. Field

The present disclosure relates to a method of culturing high-purity, highly active natural killer cells of which expression of anti-cancer-related gene is regulated, and use thereof for anticancer immunotherapy.

### 2. Description of the Related Art

Currently, the most clinically active research in the world is the anticancer immune cell therapy T-cell therapy. T-cells are the most abundant from among immune cells and are the main immune cells with high anticancer immune function. However, when allogenic T-cells provided by others are used, a severe Graft-versus host disease (GvHD) may be developed due to limitations of the major histocompatability complex (MHC).

On the other hand, natural killer cells (also referred to as "NK cells") are, from the morphological aspect, cells having large granules in the cytoplasm thereof, and account for about 5 % to 15 % of lymphocytes in the blood. Natural killer cells are immune cells that represent the primary defense (innate immune response) in the body. The natural killer cells form immune memory in the body and have fewer side effects such as cytokine storm than T-cell-based treatments that exist for a long time, and do not cause GvHD and thus can be used as an allogeneic cell therapy product. Accordingly, natural killer cells can be developed as an off-the-shelf type of treatment, and also induces antibody-dependent cell-mediated cutotoxicity (ADCC).

However, despite the therapeutic advantages and safety of natural killer cells themselves, unlike T-cells, it is difficult to culture natural killer cells. In particular, for allogeneic use, high-purity natural killer cells must be cultured without the incorporation of T-cells, but there is a limitation in that the mass proliferation of the natural killer cells is difficult to achieve. Recently, a method of mass-culturing natural killer cells using feeder cells has been developed, but when applied as a therapeutic agent, the feeder cells may be incorporated into the final product, causing safety problems.

### Detailed Description of the Invention

### Technical Problem

The inventors of the present disclosure studied a method of culturing highly pure, highly active natural killer cells, during which they found that when CD3-negative and CD56-positive natural killer cells were extracted using leukapheresis and a closed system and then IL-2, anti-NKp46 antibody, and donor plasma were used for the culturing without the use of the feeder, natural killer cells with higher cell proliferation and killing power were able to be produced than when conventional natural killer cell production methods were used.

One aspect provides a method of culturing natural killer cells, the method including:
1) selecting mononuclear cells from human peripheral blood through leukapheresis;
2) obtaining CD3-CD56+ natural killer cells from the selected mononuclear cells; and
3) treating the obtained CD3-CD56+ natural killer cells with IL-2, anti-NKp46 antibody, and plasma, followed by culturing in a feeder cell-free culture medium.

Another aspect provides natural killer cells cultured by the method or a cell population thereof.

Another aspect provides a composition for culturing natural killer cells including IL-2, anti-NKp46 antibody, and plasma, wherein
the composition does not include a feeder cell.

Another aspect provides a pharmaceutical composition for preventing or treating cancer, including natural killer cells cultured by the method or a cell population thereof as an active ingredient.

Another aspect provides a method of preventing or treating cancer, including administering the pharmaceutical composition to a subject.

Another aspect provides use of the composition for culturing natural killer cells for use in the manufacture of a medicament for preventing or treating cancer.

### Technical Solution to Problem

One aspect provides a method of culturing natural killer cells, the method including :
1) selecting mononuclear cells from human peripheral blood through leukapheresis;
2) obtaining CD3-CD56+ natural killer cells from the selected mononuclear cells; and
3) treating the obtained CD3-CD56+ natural killer cells with IL-2, anti-NKp46 antibody, and plasma, followed by culturing in a feeder cell-free culture medium.

Another aspect provides natural killer cells cultured by the method or a cell population thereof.

The method of culturing natural killer cells according to the present disclosure is different from other known natural killer cell culture methods, and the natural killer cells cultured according to the natural killer cell culture method according to the present disclosure: have a high natural killer cell purity of 99% or more and a proliferation fold of 300 to 350 times; show high cancer cell killing ability; and have significantly increased or decreased expression of anticancer-related genes before and after culture.

The term "Natural Killer cells" or "NK cells" are cytotoxic lymphocytes constituting a major component of the innate immune system, and are defined as large granular lymphocytes (LGL), and constitute third cells differentiated from B and T lymphocytes arising from common lymphoid progenitors (CLPs). The "natural killer cells" or "NK cells" include natural killer cells derived from any tissue source without additional modification, and may include natural killer progenitors as well as mature natural killer cells. The natural killer cells are activated in response to interferon or macrophage-derived cytokines, and natural killer cells include two types of surface receptors which are labeled with an "activation receptor" and an "inhibitory receptor", and control the cytotoxic activity of cells.

The natural killer cells may be purchased commercially or collected from humans or animals, and preferably supplied from humans in need of treatment with natural killer cells.

Natural killer cells may be generated from any source, for example, hematopoietic cells from placental tissues, placental perfusate, umbilical cord blood, placental blood, peripheral blood, spleen, liver, etc., such as hematopoietic stem or precursors, stem cells derived from placenta or umbilical cord, induced pluripotent stem cells, or cells differentiated therefrom. In addition, the natural killer cells may be supplied from any tissue source in vivo. For example, the natural killer cells may be included in blood collected from a living body. When the blood contains natural killer cells, the blood can be used as a natural killer cell source for application to the culture method of the present specification. The blood may be, for example, whole blood, umbilical cord blood, bone marrow, or peripheral blood.

The natural killer cells may be derived from, for example, peripheral blood mononuclear cells. The peripheral blood mononuclear cells may be obtained from a subject in need of treatment, that is, autologous peripheral blood mononuclear cells or allogenic cells. Accordingly, the natural killer cells may be autologous or allogenic natural killer cells.

The term "peripheral blood mononuclear cells (PBMC)" used herein refers to mononuclear cells selected from the peripheral blood of mammals, preferably humans, and mainly include immune cells such as B cells, T cells, and natural killer cells, and granulocytes such as basophils, eosinophils, and neutrophils and the like. The PBMC may be selected from peripheral blood collected from a living body by using leukapheresis or specific gravity centrifugation using Ficoll. In an embodiment, the PBMC may be selected by leukapheresis. The selection method thereof, however, is not limited thereto.

The term "leukapheresis" used herein refers to a method of selectively separating leukocytes from collected blood and returning the remaining components to the donor's blood. When this method is used, a large amount of mononuclear leukocytes may be obtained. Leukocytes selected by this method can be used in the present disclosure without a separate method such as the Ficoll-Hypaque density gradient method. Methods and equipment for performing leukapheresis are known in the art. Examples of leukapheresis devices include the COBESpectra^{™} manufactured by GAMBRO BCT and the CS3000 Plus Blood Cell Separator manufactured by Baxter Fenwal.

In an embodiment, the PBMCs of the present disclosure may be selected by performing leukapheresis. In the culture method according to one aspect, monocytes are selected through leukapheresis, and then CD3-CD56+ natural killer cells are obtained and cultured therefrom, thereby culturing natural killer cells with higher enrichment than in the case of culturing natural killer cells selected from whole blood. For this reason, the leukapheresis may affect the purity, activity, and expression of anticancer-related genes of natural killer cells.

The term "closed system" used herein refers to any closed system that reduces the risk of cell culture contamination during the performance of a culture process such as the introduction of new materials and during the performance of a cell culture step such as proliferation, differentiation, activation and/or selection of cells. Such systems are operated under GMP or GMP-like conditions ("sterile") to provide clinically applicable cell compositions. An example of the closed system herein is CliniMACS Prodigy^{®} (Mailenyi Biotec GmbH, Germany). This system is disclosed in WO2009/072003. However, the present disclosure is not limited to CliniMACS Prodigy^{®}.

In an embodiment, the CD3-CD56+ natural killer cell of present disclosure may be obtained by applying the CliniMACS Prodigy^{®} system on PBMC selected by leukapheresis.

The term "positive or +" used herein may refer to a case where, in relation to the cell label, a label is present in a greater amount or higher concentration when compared to other cells as a standard. That is, regarding a cell, a label is present inside or on the surface of the cell. Accordingly, when the cell can be distinguished from one or more other cell types by using the label, the cell is positive with respect to the label. Also, that could mean that a cell has a label in such an amount that is sufficient to generate a signal greater than the background value, for example, a signal of a cytometry device. For example, a cell may be labeled to be detectable by using an antibody specific for NKp44, and when the signal from the antibody is detectably greater than a control (for example, background value), the cell is "NKp44+." The term "negative or -" refers to a case where, even when an antibody specific to a specific cell surface label is used, the label cannot be detected compared to the background value.

In an embodiment, the natural killer cells cultured according to the method may be natural killer cells of which the activity of at least one factor selected from the group consisting of immune activation receptors, apoptosis inducers (cancer cells), anti-apoptosis inducers (natural killer cells), chemokines, chemokine receptors, immune enhancing factors, and anticancer cofactors is increased, and of which the activity of at least one selected from the group consisting of immune inactivation receptors, immunosuppressive factors, and apoptosis inducers (natural killer cells) is reduced. The term "anti-cancer-related gene" used herein refers to a gene encoding listed anti-cancer-related factors.

In an embodiment, the natural killer cells may refer to cells of which the cytotoxic or natural killer cell's natural immunomodulatory ability is activated compared to parental cells, such as hematopoietic cells or natural killer progenitors, or cells of the expression of anticancer-related factors described above is regulated. In a specific embodiment, the natural killer cells may be CD3-CD56+.

In another embodiment, the expression of the immune activation receptor may be expression of one or more selected from the group consisting of NKG2D, NKp30, NKp44, and NKp46.

In another embodiment, the expression of the immune inactivation receptor may be expression of one or more selected from the group consisting of KIR3DL2 and Siglec9.

In another embodiment, the expression of the apoptosis inducer may be the expression of FASL and/or TRAIL (TNFSF10).

In another embodiment, the expression of the apoptosis inducer may be the expression of one or more selected from the group consisting of Granzyme A, Granzyme K, and Perforin.

In another embodiment, the expression of the chemokine and/or chemokine receptor may be the expression of CCL1 and/or CCR5.

In another embodiment, the anticancer cofactor may be expression of DAP10 (HCST) and/or IL-2RA.

In another embodiment, the immune enhancing factor may be the expression of GM-CSF.

In another embodiment, the immunosuppressive factor may be an expression of IL-8 (CXCL8) and/or IL-10.

In another embodiment, the apoptosis inducer (natural killer cells) may be an expression of Noxa.

In another embodiment, the anti-apoptosis inducer (natural killer cells) may be an expression of BCL-2.

Natural killer cells cultured by the culture method according to one aspect may have one or more characteristics selected from the following (a) to (e) compared to natural killer cells before culture:
(a) an increase in the expression of one or more selected from the group consisting of NKG2D, NKp30, NKp44, and NKp46;
(b) a decrease in the expression of one or more selected from the group consisting of KIR3DL2 and Siglec9;
(c) an increase in the expression of one or more selected from the group consisting of FASL and TRAIL (TNFSF10);
(d) an increase in the expression of one or more selected from the group consisting of Granzyme A, Granzyme K, and Perforin; and
(e) an increase in the expression of one or more selected from the group consisting of CCL1 and CCR5.

In addition, the natural killer cells cultured according to the method may additionally have one or more characteristics selected from the following (f) to (j) compared to the natural killer cells before culture:
(f) an increase in the expression of one or more selected from the group consisting of DAP10 (HCST), and IL-2RA;
(g) an increase in the expression of GM-CSF (CSF2);
(h) a decrease in the expression of one or more selected from the group consisting of IL-8 (CXCL8), and IL-10;
(i) a decrease in the expression of Noxa (PMAIP1); and
(j) an increase in the expression of BCL-2.

In an embodiment, in the natural killer cells cultured according to the above method, the expression of activation receptors is significantly increased and the expression of inactivation receptors is significantly decreased. Accordingly, the natural killer cells have higher purity, greater cell proliferation fold, and stronger cancer cell killing ability than natural killer cells before culture.

The term "activating" or "activation receptor" used herein refers to a molecule on the surface of natural killer cells that causes a measurable increase in any property or activity that is known in the art and related to the activity of natural killer cells, for example, an ability to stimulate cytokine (for example, IFN-γ and TNF-α) production, an increase in intracellular free calcium, for example, lysis ability with respect to target cell, or the proliferation of natural killer cells, when stimulated.

Examples of the activation receptor of natural killer cells include, but are not limited to, NKG2D, NKp30, NKp44, NKp46, IL-12R, IL-15R, IL-18R, or IL-21R.

The activation receptors of natural killer cells mainly recognize ligands whose expression increases when target cells are in an abnormal state, and cause cytotoxic effects to eliminate target cells.

NKG2D provides cytotoxic activity by detecting UL16 binding proteins (ULBPs), MIC A/B, RAE1, H60, and MULT1, which are intracellular molecules whose expression is increased during DNA damage, cancer development, and viral infection.

NKp30 is a receptor activated by the binding of extracellular ligands including BAG6, NCR3LG1, and B7-H6, and binds to these ligands to stimulate cytotoxicity.

NKp44 recognizes, as ligands, glycoproteins of the cell surface and proteoglycans, nuclear proteins that can be exposed outside the cell, and molecules that are released into the extracellular space or migrate into extracellular vesicles. Recently, NKp44 has been reported to recognize soluble plasma proteins (for example, PDGF-DD) such as extracellular matrix (ECM)-derived glycoproteins or growth factors (Cell. 2018 January 25; 172 (3): 534-548.e19., Front Immunol. 2019; 10:719.).

The term "inactivating" or "inactivation receptor" used herein refers to a molecule on the surface of natural killer cells that measurably reduce certain properties or activities that is known in the art and related to the activity of natural killer cells, for example, an ability to produce cytokines (for example, IFN-γ and TNF-α), increase in intracellular free calcium, for example, lysis of target cells, for example, an ability to lyse target cells, when stimulated.

Examples of the natural killer cell inactivation receptor include KIR2DL1, KIR2DL2/3, KIR2DL4, KIR2DL5A, KIR2DL5B, KIR3DL1, KIR3DL2, KIR3DL3, LILRB1, NKG2A, NKG2C, NKG2E, Sigleg9 (sialic acid binding Ig-like lectin 9), or LILRB5, and are not limited to.

FASL and TRAIL are factors involved in cytotoxicity and immune activation of natural killer cells, and are representative proteins that bind to receptors on the cell surface and induce apoptosis of target cells.

Regarding the natural killer cells according to one aspect, the expression of NKp44 and NKp30 is significantly increased by 1064 and 5.2 times, respectively, the expression of KIR3DL2 and Siglec9 is significantly decreased, and the expression of FAS-L (FASLG) and TRAIL (TNFSF10) is increased remarkably from 6.1 times to 24.5 times. As such, the natural killer cells have high anticancer effects and thus can be used as an effective cell therapy.

T cells are largely classified as CD8-positive cytotoxic T cells (Tc) and CD4-positive helper T cells (Th), and cytokines secreted from the Th cells are divided into Th1 type and Th2 type. Th1 cells are mainly involved in intracellular defense by phagocytosis, and mainly produce cytokines such as IL-2, IL-12, INF-γ, or TNF-α.

Meanwhile, Th2 cells mainly produce cytokines such as IL-4, IL-6, IL-8, or TGF-β, suppressing T cell activity and suppressing the production of Th1 type cytokine. Thus, activation of Th1 cells and suppression of Th2 cells may affect anticancer immune functions.

Among them, IL-10 is produced in Th2 cells and interferes with the production of cytokine in Th1 cells.

IL-8 (CXCL8) is a pro-inflammatory cytokine that mainly mediates the inflammatory response, and is a representative chemokine that attracts, to the site of inflammation, a neutrophil that promotes the proliferation of cancer cells and neoangiogenesis by combining with CXCR1/2 of tumors.

Regarding natural killer cells according to one aspect, the expressions of IL-10 and IL-8, which inhibit anticancer immune function, are significantly reduced by 1/100 and 1/1000 times, respectively, and thus, the natural killer cells are expected to be used as an efficient cell therapy.

The term "chemokine" used herein is a substance secreted from activated natural killer cells, and chemokine can mobilize (or migrate) various inflammatory cells to target organs. Chemokines are classified as four groups (C, CC, CXC, and CX3X groups) according to the arrangement structure of the first two N-terminal cysteines in the amino acid sequence. As of now, more than 40 chemokines and more than 20 chemokine receptors are known. In particular, in the tumor microenvironment consisting of the tumor and the cells of the host surrounding the tumor, tumor-associated host cells and cancer cells secrete a variety of chemokines, and accordingly, various types of cells mediating the balance between anti-tumor and tumor-promoting responses, are recruited and activated. Furthermore, in addition to the involvement in chemotaxis, chemokines are also involved in other tumor-related processes including tumor cell growth, angiogenesis and metastasis.

In an embodiment, the chemokine receptor may be CCR or CXCR, and CCR may be CCR1, CCR2, CCR2B, CCR3, CCR4, CCR5, CCR6, CCR7, CCR8, CCR9, or CCR10, for example, CCR2, CCR5, or CCR6. In addition, the chemokine may be CCL1, CCL3, CCL4, CCL5, CCL22, or CXCL8, for example, CCL1.

The term "CD3-CD56+ natural killer cell" used herein may be interchangeably used with "CD3-CD56+ NK cell" or "CD3-/CD56+ NK cell".

The CD3-CD56+ natural killer cells may be in such a state as being thawed after cryopreservation. In an embodiment, mononuclear cells of the peripheral blood are selected from blood, frozen, and then thawed to be used in culturing of natural killer cells. The freezing may be performed using a conventionally known method, for example, a freezing method including a first freezing step in the temperature range of 4 °C to - 42 °C, a second freezing step in the temperature range of -42 °C to -15 °C, and a third freezing step in a range of -15 °C to -120 °C. In each freezing step, the temperature may be raised or decreased to the upper or lower limit of the temperature range of each freezing step by sequentially maintaining a sample at various discontinuous temperatures within the temperature range for a certain time period. In an embodiment, the first freezing step may be performed at 0 °C for 10 minutes to 15 minutes, at - 12 °C for 5 minutes to 10 minutes, and at -42 °C for 0.5 minutes to 1 minute, the second freezing step may be performed, after the first freezing step, at -25 °C for 1 minute to 3 minutes and at -15 °C for 1 minute to 3 minutes, and the third freezing step may be performed, after the second freezing step, at -42 °C for 20 to 40 minutes and at -120 °C for 20 minutes to 50 minutes. In another embodiment, the first freezing step may be performed in the temperature range of 4 °C to -40 °C at the rate of 0.5 °C/m to 5 °C/m, the second freezing step may be performed, after the first freezing step, in the temperature range of -40 °C to - 90 °C at the rate of 1 °C/m to 10 °C/m, and the third freezing step may be performed, after the second freezing step, in the temperature range of -90 °C to -120 °C at the rate of 1 °C/m to 10 °C/m. Each freezing step of the freezing method may be performed by a controlled rate freezer (CRF). In the case of freezing peripheral blood mononuclear cells described in the present disclosure, the separated cells are suspended in a mixture of cryostor CS10 or ALyS505NK-EX and Albumin+DMSO, adjusted to be in an appropriate number of cells, and then frozen. The cryopreserved mononuclear cells of the peripheral blood may be provided for culture and proliferation of natural killer cells according to the present disclosure after thawing, and cell culture may be performed at a necessary time point through a freezing process. The present disclosure provides a method of freezing and thawing mononuclear cells of peripheral blood that can be effectively applied to the method of culturing natural killer cells which maintains a high survival rate and are activated.

According to the method of culturing natural killer cells according to one aspect, CD3-CD56+ natural killer cells may be cultured in the presence of gamma globulin (IgG) and fibronectin. The culture in the presence of gamma globulin and fibronectin may be a culturing in a culture medium including gamma globulin and fibronectin, or a culturing in a culture vessel on which gamma globulin and fibronectin are coated on the culture surface (the surface of the culture vessel that may come into contact with cells). In an embodiment, natural killer cells may be cultured in a culture vessel on which gamma globulin and fibronectin are coated. The gamma globulin and fibronectin-coated culture vessel may be prepared by adding and coating a solution containing gamma globulin and fibronectin. In addition, a known adhesive protein may be used instead of the fibronectin, and for example, collagen may be used. In an embodiment, gamma globulin and fibronectin may be coated on a culture vessel by incubating at low temperature (for example, 2 °C to 4 °C) after adding a solution containing gamma globulin and fibronectin to the culture vessel (for example, a T75 flask).

The concentration of gamma globulin may be 0.1 ng/ml to 1 ng/ml, 1 ng/ml to 10 ng/ml, 10 ng/ml to 100 ng/ml, or 1 ng/ml to 100 ng/ml. Gamma globulin may activate natural killer cells by stimulating FcγRIII of natural killer cells.

The concentration of the fibronectin may be 0.1 µg/ml to 50 µg/ml, 1 µg/ml to 50 µg/ml, 5 µg/ml to 50 µg/ml, or 10 µg/ml to 50 µg/ml. Fibronectin may facilitate cell-to-cell interactions by promoting the migration of natural killer cells.

The culturing may be culturing natural killer cells by culturing mononuclear cells of the peripheral blood.

Culturing of the mononuclear cells of the peripheral blood may be performed under normal cell culture conditions, that is, in a CO₂ incubator at about 37 °C, and may be continuously cultured while adding a culture medium once every 2 or 3 days. In addition, the concentration of PBMC added to the medium may be in the range of 4 × 10⁵ cells/ml to 5 × 10⁷ cells/ml, but is not limited thereto. The culture period may be, for example, 3 days to 28 days, 5 days to 25 days, 7 days to 23 days, 10 days to 18 days, or 10 days to 14 days, but is not limited thereto. For example, the culture period may be appropriately set within or outside the culture periods to obtain the desired number of natural killer cells. The culture vessel may include commercially available dishes, flasks, plates, multi-well plates, and culture bags.

On the other hand, in order to proliferate the natural killer cell to the maximum, the culture may be cultured at each stage. In this case, culture steps may have the same or different culture medium components, culture vessels, and culture periods, and the proliferation of natural killer cells may be achieved by finding the optimal culture period for each step. In one or more embodiments of the present disclosure, during the culture period, a culture composition (or culture medium) is added to a flask every 2 to 3 days, in order to help the effectively culturing of natural killer cells.

Another aspect provides a composition for culturing natural killer cells including IL-2, anti-NKp46 antibody, and plasma, wherein

The composition may be a composition that does not include a feeder cell.

According to the method of culturing natural killer cells according to one aspect, CD3-CD56+ natural killer cells may be treated with IL-2, anti-NKp46 antibody, and plasma, and then cultured in a feeder cell-free culture medium. The culture medium may be a immune cell culture medium of the related art including IL-2, an anti-NKp46 antibody, and plasma. Examples of the immune cell culture medium are ALyS505NK-EX (Cell Science & Technology Inst. Inc., Japan), RPMI1640 (Life technologies, USA), x-vivo10 (Lonza, USA), CellGro SCGM (CellGenix, Germany), KBM (Kohjin bio, Japan), etc., and are not limited thereto.

The concentration of IL IU/mL to 2 contained in the culture medium may be 500 IU/mL to 5,000 IU/mL, 600 IU/mL to 4,000 IU/mL, 700 IU/mL to 3,000 IU/mL, 800 IU/mL to 2,000 IU/mL, 900 IU/mL to 1,500 IU/mL, 900 lU/mL to 1,200

IU/mℓ, or 1,000 IU/mL to 1,200 IU/mℓ, and is not limited thereto. The IL-2 may promote the growth of natural killer cells.

The concentration of the anti-NKp46 antibody contained in the culture medium may be 0.1 µg/ml to 10 µg/ml, 0.5 µg/ml to 10 µg/ml, 1 µg/ml to 10 µg/ml, or 5 µg/ml to 10 µg/ml, but is not limited thereto. NKp46 is an activation receptor for natural killer cells, and according to, the anti-NKp46 antibody may stimulate NKp46 to activate natural killer cells.

In addition, the plasma included in the culture medium may be obtained from peripheral blood from which mononuclear cells of the peripheral blood used for culturing natural killer cells are selected. Serum may be used instead of the plasma.

The plasma concentration may be 1 v/v% to 20 v/v%, 1 v/v% to 15 v/v%, 2 v/v% to 15 v/v%, 5 v/v% to 15 v/v%, or 5 v/v% to 10 v/v%, based on the total culture medium, and is not limited thereto.

The composition for culturing natural killer cells according to one aspect does not include feeder cells. Conventionally, natural killer cells are cultured by using a method of increasing the proliferation rate and cytotoxicity using cancer cell lines as feeder cells. However, when cancer cell lines are as feeder cells, it is difficult to ensure safety, which is an important factor considered, during clinical application. Also, the cultured natural killer cells are only natural killer cells that are endowed with priming specificity for specific cancer cells. In addition, according to the present disclosure, complexity of the process of proving that feeder cells are completely removed after culture is removed, and by excluding the possibility of contamination of feeder cells, the safety can be further enhanced.

In one embodiment, the composition according to the aspect does not use feeder cells, and contains only IL-2, anti-NKp46 antibody, and donor plasma. Even when CD3-CD56+ natural killer cells are cultured in vitro for a long period of time by using the same, a stable culture may be obtained in which the cell proliferation and the cell viability are at remarkably high levels. In addition, the natural killer cells exhibit significantly increased cancer cell killing ability, and also, since feeder cells are not included, they can be safely applied clinically compared to existing natural killer cells.

The culture medium in each culture step may contain appropriate proteins, cytokines, antibodies, compounds and other components, provided that the effect of increasing the purity, proliferation fold, and cancer cell killing ability of natural killer cells is not decreased.

Natural killer cells cultured using the composition may have one or more characteristics selected from the following (a) to (e) compared to natural killer cells before culture:
(a) an increase in the expression of one or more selected from the group consisting of NKG2D, NKp30, NKp44, and NKp46;
(b) a decrease in the expression of one or more selected from the group consisting of KIR3DL2 and Siglec9;
(c) an increase in the expression of one or more selected from the group consisting of FASL and TRAIL (TNFSF10);
(d) an increase in the expression of one or more selected from the group consisting of Granzyme A, Granzyme K, and Perforin; and
(e) an increase in the expression of one or more selected from the group consisting of CCL1 and CCR5.

In addition, the natural killer cells cultured according to the method may additionally have one or more characteristics selected from the following (f) to (j) compared to the natural killer cells before culture:
(f) an increase in the expression of one or more selected from the group consisting of DAP10 (HCST), and IL-2RA;
(g) an increase in the expression of GM-CSF (CSF2);
(h) a decrease in the expression of one or more selected from the group consisting of IL-8 (CXCL8), and IL-10;
(i) a decrease in the expression of Noxa (PMAIP1); and
(j) an increase in the expression of BCL-2.

In an embodiment, the plasma may be separated from the human peripheral blood.

Also, in an embodiment, the composition may further include gamma globulin and fibronectin.

According to the present disclosure, regarding the expression, the listed anticancer-related genes or factors may be more or less expressed after culture than in the natural killer cell or natural killer cell population before culture. In addition, for example, the cultured natural killer cells or natural killer cell population express at least 70% or more, at least 80% or more, at least 90% or more, at least 95% or more, It may express at least 96% or more, at least 97% or more, or at least 99% or more, or 100% of the factor, based on the total cells or the total cell population. The "% expression" or "expression rate" may refer to the percentage of natural killer cells expressing one or more of the factors relative to the total number of cells in a sample. The expression or expression rate of the factor in the cell or cell population may be determined by a value obtained by detection using a flow cytometer.

In another embodiment, the present disclosure may provide a method further including the step of suspending the natural killer cells cultured by the natural killer cell culture method in a solution containing dextran and albumin and storing the natural killer cells.

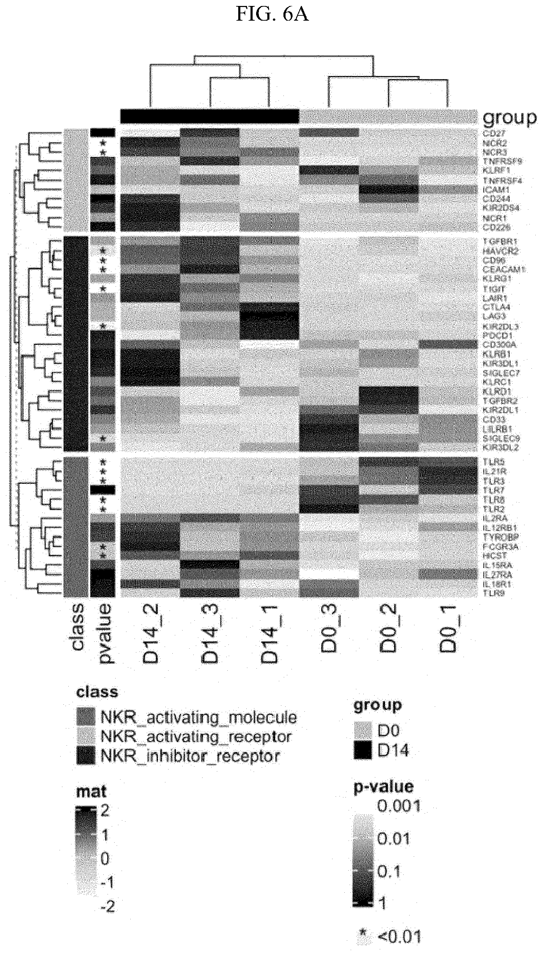

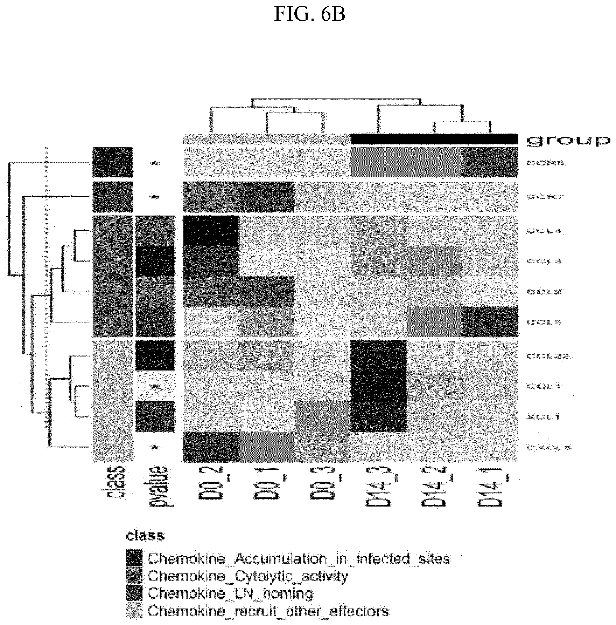

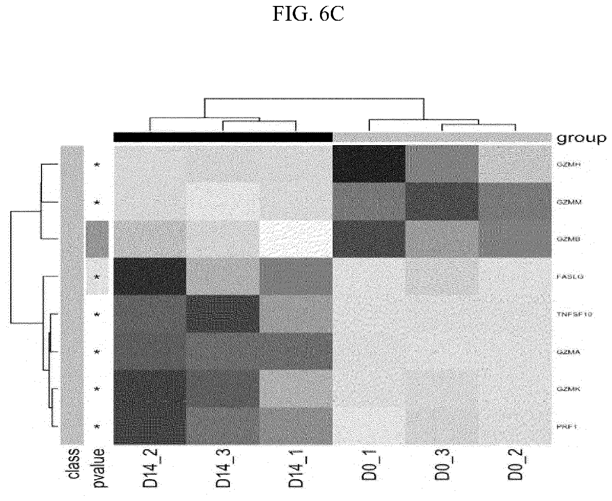

In an embodiment, the natural killer cell cultured by the natural killer cell culture method of present disclosure showed, compared to before culture, a high purity of 99% or more, a proliferation fold of 300 to 350 times, and a high cancer cell killing titer, a significant increase in the expression of factors associated with cell killing power, a significant increase in the expression of chemokine receptors that recruit inflammatory effector cells, a significant decrease in the expression of inactivation receptors that inactivate natural killer cells when combined with ligand, a significant increase in genes that inhibit apoptosis of natural killer cells, and a significant decrease in genes that promote the same (see FIG. 6 and Table 1).

According to the present disclosure, the natural killer cell may be cultured or genetically engineered to express the anticancer-related factors or to increase or decrease the expression or activity thereof.

According to the present disclosure, the culture may be performed such that the expression or activity of the anticancer-related factors described above is increased or decreased using any source, for example, hematopoietic cells from placental tissues, placental perfusate, umbilical cord blood, placental blood, peripheral blood, spleen, liver, etc., such as hematopoietic stem or precursors.

The term "genetic engineering" or "genetically engineered" refers to the act of introducing one or more genetic modifications to a cell or a cell produced thereby.

The term "increase in activity" or "increased activity" refers to a detectable increase in the activity of a protein or enzyme. The term "increase in activity" or "increased activity" refers to a higher level of activity of a protein or an enzyme, compared to a given parental or wild-type cell, or cells before culture (for example, PBMC).

Another aspect provides a cell therapy or a pharmaceutical composition, including natural killer cells cultured by the method or a cell population thereof as an active ingredient.

The term "cell therapy" used herein refers to a medicament product used for the purpose of treatment, diagnosis, and prevention through a series of actions such as changing the biological characteristics of cells through the proliferation/selection of living autologous, allogenic, and xenogenic cells in vitro to restore cell and tissue functions or other methods. The United States and Korea have been managing cell therapy products as pharmaceuticals since 1993, and 2002, respectively. These cell therapies can be broadly classified as two fields. The first is stem cell therapy for tissue regeneration or organ function recovery, and the second is immune cell therapy for regulating immune response, such as suppression of immune response in vivo or enhancement of immune response.

The cell therapy or pharmaceutical composition may be for preventing or treating cancer or infectious diseases.

Another aspect provides use of the natural killer cell or the cell population thereof for the manufacture of medicament.

Another aspect provides a method of treating a disease including administering the natural killer cells or cell population thereof to a subject.

The term "disease" used herein may refer to a certain pathological condition, in particular, cancer, infectious disease, inflammatory disease, metabolic disease, autoimmune disorder, degenerative disease, apoptosis-related disease, and graft rejection.

The term "infectious disease" used herein may collectively refer to infectious diseases caused by organisms such as bacteria, viruses, and fungi. For example, in the case of viral infections such as human immunodeficiency virus (HIV), epstein-barr virus (EBV), human herpes virus (HHV), and influenza A virus (IAV), activation receptors, such as NKp46, NKp44, or NKp30, of which expression is increased in the natural killer cells, may bind to and activate viral glycoproteins to induce death of infected cells. In addition, for example, in the case of viral infections such as cytomegalovirus (CMV) and encephalomyocarditis virus (EMCV), death receptors, such as FASL, TRAIL, etc., may bind to viral glycoproteins of the virus and be activated to induce death of infected cells.

The term "treatment" used herein refers to, or includes, the alleviation, inhibition of progression, or prevention of a disease, disorder or condition, or one or more symptoms thereof, and the term "active ingredient" or "pharmaceutically effective amount" refers to any amount of a composition used in the practice of the invention provided herein sufficient to alleviate, inhibit the progression of, or prevent a disease, disorder or condition, or one or more symptoms thereof.

The terms "administering," "introducing," and "implanting" used herein, are interchangeably used, and may refer to the arrangement of a composition according to an embodiment into a subject through a method or route of causing at least partial localization of the composition according to an embodiment to a desired site. The administration may be performed through an appropriate route through which at least a portion of the cells or cellular components of the composition according to an embodiment is delivered to a desired location within a living subject. The survival period of cells after the administration to a subject, may be as short as several hours, for example, 24 hours to several days, and as long as several years.

The administration may be administered in combination with an additional anticancer agent or a cytokine agent. Examples of additional anticancer agents include alkylating agents, antimetabolites, spindle inhibitor plant alkaloids, cell disfunctional/antitumor antibiotics, topoisomerase inhibitors, antibodies, photosensitizers, and kinase inhibitors. Examples of the anticancer agent may include compounds used in targeting therapy and conventional chemotherapy. In addition, examples of the antibody include alemtuzumab, apolizumab, acelizumab, atlizumab, bafinuzumab, bevacizumab, vibatuzumab mertansine, cantuzumab mertansine, cedelizumab, certolizumab pegol, cidfushituzumab, cidtuzumab, daclizumab, eculizumab, efalizumab, efratuzumab, erlizumab, felbizumab, pontolizumab, gemtuzumab ozogamicin, inotuzumab ozogamicin , ipilimumab, labetuzumab, lintuzumab, matuzumab, mepolizumab, motavizumab, motobizumab, natalizumab, nimotuzumab, nolovizumab, numavizumab, ocrelizumab, omalizumab, palibizumab, pascolizumab, pexituzumab, pectuzumab, pertuzumab, pexelizumab, lalibizumab, ranibizumab, reslibizumab, reslibizumab, resaibizumab, lovelizumab, ruplizumab, sibrotuzumab, ciplizumab, sontuzumab, tacatuzumab tetraxetan, tadocizumab, talizumab, tefibazumab, tocilizumab, toralizumab, trastuzumab, tucotuzumab selmolyukin, tucucitu zumab, umabizumab, urtoxazumab, and visilizumab.

The term "selected cell", for example, "selected natural killer cell" used herein, refers to a tissue from which a cell originates, for example, a cell substantially selected from the peripheral blood.

A composition according to one aspect may be used to treat or prevent a tumor or cancer derived from a neoplasm. Neoplasms may be malignant or benign, cancers may be primary or metastatic, a neoplasm or cancer may be in an early or late stage. Non-limiting examples of neoplasms or cancers that can be treated, include at least one selected from the group consisting of glioma, gastrointestinal stromal tumor, leukemia, breast cancer, uterine cancer, cervical cancer, gastric cancer, colon cancer, prostate cancer, ovarian cancer, lung cancer, laryngeal cancer, rectal cancer, liver cancer, gallbladder cancer, pancreatic cancer, prostate cancer, kidney cancer, skin cancer, bone cancer, muscle cancer, fat cancer, fibrocyte cancer, hematological cancer, lymphoma, and multiple myeloma, and may include, for example, at least one selected from the group consisting of glioma, gastrointestinal stromal tumor, leukemia, breast cancer, uterine cancer, cervical cancer, gastric cancer, colon cancer, prostate cancer, and ovarian cancer. The glioma may be glioma, and the leukemia may be chronic myelomonocytic leukemia.

In an embodiment, natural killer cells according to one aspect are expected to be more effective in carcinomas which highly express NKp44 ligands, such as MML5, HSPG, BAG6, and B6-H6, and NKp30 ligands. Tumors in which PDGF is increased, include glioma, gastro-intestinal stromal tumor, chronic myelomonocytic leukemia, and breast cancer. In particular, PDGFα is reported to be highly correlated with lymph node metastasis of breast cancer, high histologic grade, ER/PR negativity, and triple-negative breast subtype (Breast Cancer Res Treat. 2018; 169(2): 231-241). Therefore, the natural killer cells according to one aspect may be more effective against these tumors.

In addition, in an embodiment, in natural killer cells according to one aspect, CCL1 (318 times) was significantly increased from among chemokines, and CCR5 (49.9 times) was significantly increased from among chemokine receptors. Immune cells of which expression is increased, may recruit other effector cells such as monocytes, B-cells, and dendritic cells and are more advantageous in killing tumor cells. In particular, when the expression of CCR5 is increased, the cells may have affinity for CCL3/CCL4/CCL5+ tumor cells, thereby being more advantageous for killing these tumors. CCL5 is highly expressed in metastatic/advanced breast cancer, cervical cancer, gastric cancer, colon cancer, prostate cancer, ovarian cancer (Mediators of Inflammation Volume 2014, Article ID 292376), etc. Accordingly, natural killer cells according to an aspect, having increased CCR5, may be more effective against the tumor.

In an embodiment, the method of administering the pharmaceutical composition is not particularly limited, and may be administered orally or parenterally, such as intravenous, subcutaneous, intraperitoneal, inhalation or topical application, depending on the desired method. The dosage varies depending on the patient's weight, age, sex, health condition, diet, administration time, administration method, excretion rate, and severity of the disease. A daily dosage refers to an amount of a therapeutic substance according to one aspect sufficient to treat a disease state alleviated by administration to a subject in need thereof. An effective amount of a therapeutic agent vaies depending on the particular compound, the disease state and the severity thereof, and the subject in need of treatment, and may be routinely determined by one of ordianry skilled in the art. As a non-limiting example, the dosage of the composition according to one aspect to the human body may vary depending on the patient's age, weight, sex, dosage form, state of health, and degree of disease. Regarding an adult patient weighing 70 kg, the dosage may be administered in divided doses once or several times a day at regular time intervals, or may be administered several times at regular time intervals. For example, the dosage may be administered in divided doses at about 1,000 cells/time to about 10,000 cells/time, about 1,000 cells/time to about 100,000 cells/time, about 1,000 cells/time to about 1,000,000 cells/time, about 1,000 cells/time to about 10,000,000, about 1,000 cells/time to about 100,000,000 cells/time, about 1,000 cells/time to about 1,000,000,000 cells/time, about 1,000 cells/time to about 10,000,000,000 cells/time, or about or 1,000 cells/time to about 100,000,000,000 cells/circuit.

The term "subject" used herein refers to a subject in need of treatment for a disease, and more specifically, refers to primates that are human or non-human, and mammals such as mice, rats, dogs, cats, horses, and cattle.

A pharmaceutical composition according to an embodiment may include a pharmaceutically acceptable carrier and/or additives. For example, sterile water, physiological saline, common buffers (phosphoric acid, citric acid, other organic acids, etc.), stabilizers, salts, antioxidants (ascorbic acid, etc.), surfactants, suspending agents, isotonic agents, or preservatives, etc. may be included. For topical administration, the pharmaceutical composition may include the combination with organic substances, such as biopolymers, or inorganic substances, such as hydroxyapatite, specifically collagen matrices, polylactic acid polymers or copolymers, polyethylene glycol polymers or copolymers, and chemical derivatives thereof. When the pharmaceutical composition according to an embodiment is prepared in a formulation suitable for injection, the immune cells or substances that increase the activity thereof may be dissolved in a pharmaceutically acceptable carrier or frozen in a dissolved solution state.

The pharmaceutical composition according to an embodiment, if needed according to the administration method or dosage form, may appropriately include a suspending agent, a solubilizing agent, a stabilizer, an isotonic agent, a preservative, an anti-adsorption agent, a surfactant, a diluent, an excipient, a pH adjuster, an analgesic agent, a buffer, a reducing agent, an antioxidant, and the like. Pharmaceutically acceptable carriers and agents suitable for the present disclosure, including those exemplified above, are described in detail in Remington's Pharmaceutical Sciences, 19th ed., 1995. The pharmaceutical composition according to an embodiment may be formulated in unit dosage form by using a pharmaceutically acceptable carrier and/or excipient or prepared by being inserted into a multi-dose container, according to a method that can be easily performed by those skilled in the art. The dosage form may be in the form of a solution in an oil or aqueous medium, suspension or emulsion , or in the form of powder, a granule, a tablet, or a capsule.

Another aspect provides use of natural killer cells cultured by the method or cell populations thereof.

The use of a pharmaceutical composition including natural killer cells cultured by the method or cell populations thereof as an active ingredient is provided.

Another aspect provides the use of the composition for culturing natural killer cells for use in the manufacture of a pharmaceutical composition or preparation for preventing or treating cancer.

Another aspect provides use of the composition for culturing natural killer cells for use in the manufacture of a medicament for preventing or treating cancer.

The natural killer cells, pharmaceutical compositions, cancer, prevention, treatment, etc. are as described above.

### Advantageous Effects of Disclosure

In the natural killer cells according to one aspect, the expression of anticancer related genes is significantly increased or decreased before and after culture. The natural killer cells have the unique characteristics thereof, and remarkably high activity and proliferation fold. Therefore, the natural killer cells can be used for effective anticancer immunotherapy.

### Brief Description of Drawings

FIG. 1A is a diagram showing the results of FACS analysis of the phenotype of Donor 1 natural killer cells on the 21st day after culture.
FIG. 1B is a diagram showing the results of FACS analysis of the phenotype of Donor 2 natural killer cells on the 21st day after culture.
FIG. 2 is a diagram showing the change in the number of cells for each culture period of natural killer cells according to an embodiment.
FIG. 3 is a diagram showing the change in the proliferation fold on the 21st day after culturing natural killer cells according to an embodiment.
FIG. 4 is a diagram showing the change in viability of natural killer cells for each culture period of natural killer cells according to an embodiment.
FIG. 5 is a diagram showing the change in cell killing ability according to the E:T ratio of natural killer cells according to an embodiment.
FIG. 6A is a heat map showing the change in clustering of natural killer cells according to the change in natural killer cell receptor-related genes according to an embodiment.
FIG. 6B is a heat map showing the change in the clustering according to the change in cytokine/chemokine genes of natural killer cells according to an embodiment.
FIG. 6B is a heat map showing the change in the clustering according to the change in cell killing-related genes of natural killer cells according to an embodiment.

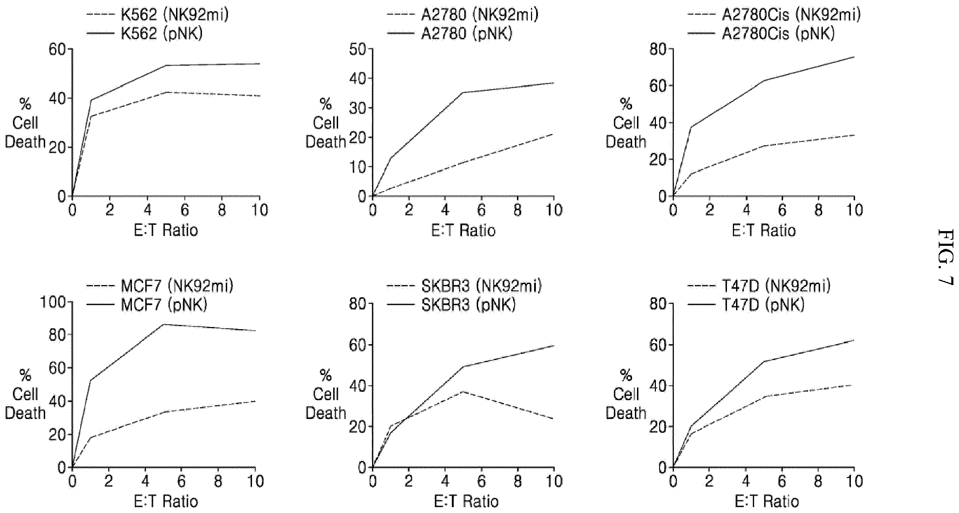

FIG. 7 is a diagram in which changes in cell killing ability according to the E:T ratio of natural killer cells according to an embodiment and a representative human NK cell line are compared.

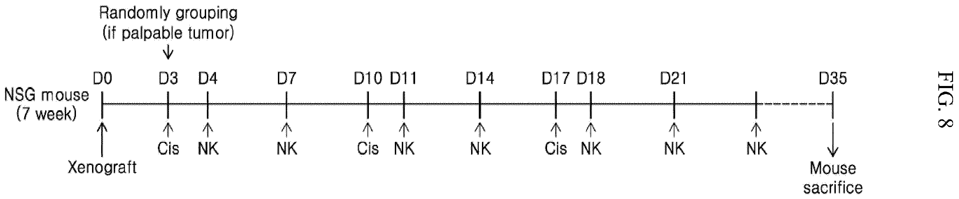

FIG. 8 is a diagram showing an administration schedule for confirming an anticancer effect according to administration of natural killer cells according to an embodiment.

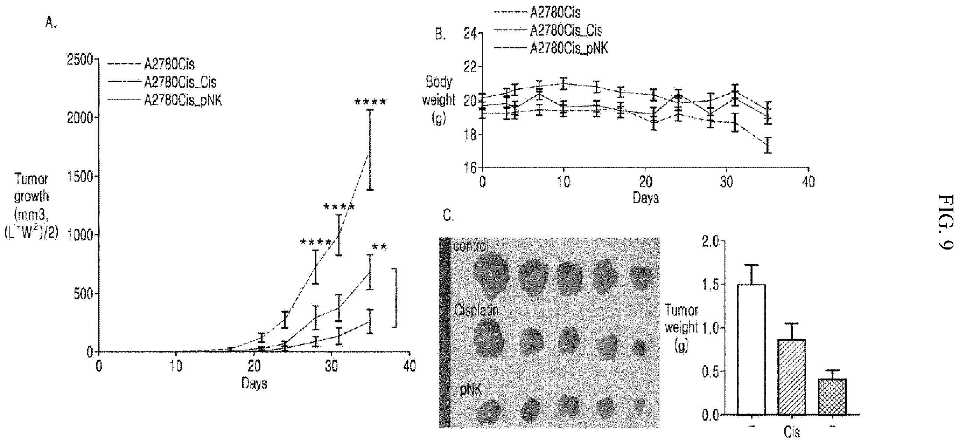

FIG. 9 is a view showing the results of confirming the anticancer activity of natural killer cells in an ovarian cancer mouse model according to an embodiment.

### Mode of Disclosure

Hereinafter, the present disclosure will be described in more detail through examples. However, these examples are provided herein for illustrative purpose only, and the scope of the present disclosure is not limited to these examples.

### Example. Manufacturing of highly pure, highly active natural killer cells in which the expression of anticancer-related genes is regulated

To prepare highly pure, highly active natural killer cells in which anticancer-related gene expression is regulated, natural killer cells were cultured as follows.

### 1. Manufacture of natural killer cells

### 1.1. Selection of CD3-CD56+ natural killer cells

Peripheral blood mononuclear cells (PBMC) and plasma (300 ml) of three healthy donors (Donor 1, Donor 2, and Donor 3) were each separated by leukapheresis, and CD3-CD56+ natural killer cells were obtained from 2 to 5 × 10¹⁰(cells) of selected monocytes by using the CliniMACS Prodigy^{®}system. Specifically, the leukapheresis was performed using Spectra-Optia (Ver. 11) component collection equipment, and 14000 mL to 16000 mL of blood was circulated using 500 mL of heparin 3000 U/ACD (acid citrate dextrose). The final collected amount was 150 mL to 250 mL, and the total number of white blood cells collected was 2 × 10¹⁰ (cells) to 5 × 10¹⁰ (cells). Next, preparation of CD3-CD56+ natural killer cells was performed using the closed system CliniMACS Prodigy^{®}. The T cell fraction (%) was calculated using the leukocytes in the collected blood as a starting material, and then, the number of T cells was calculated with respect to the total volume, and the total number of T cells was adjusted to be 9.5 × 10⁹ (cells). Anti-CD3 monoclonal antibody-conjugated microbeads (7.5 mL/95 mL buffer) were put into the cell culture chamber and a reaction was caused to occur to obtain CD3-negative cells after 4-5 hours (0.9 to 1 × 10¹⁰ cells), and then the CD3-negative cells were positively selected using anti-CD56-conjugated microbeads (7.5 mL/95 mL buffer) to obtain CD3-CD56+ natural killer cells in the amount of 0.3 to 3 × 10⁹ (cells).

### 1.2. Freezing and thawing of CD3-CD56+ natural killer cells

The CD3-CD56+ natural killer cells selected in Example 1.1 were frozen at 3 × 10⁸ (cells)/vial, and a total of 3 vials were transferred to an LN2 tank and stored (-130 °C or lower). Thereafter, when needed for experiments, the cells were thawed before used.

Specifically, all cell suspensions obtained in Example 1.1 were centrifuged at 1,500 rpm for 5 minutes, and the supernatant was removed therefrom. The natural killer cells obtained were suspended using Cryostor CS10 or ALyS505NK-EX+Albumin+DMSO mixture stored at 2 °C to 8 °C to make the number of cells to be 1 Y10⁶ cells/ml to 100 Y10⁶ cells/ml. The suspended cells were dispensed into 2 ml cryogenic vials by 1 ml for each, and then, subjected to, using a controlled rate freezer (CRF), : a first freezing step in which the freezing was performed at 0 °C for 10 minutes to 15 minutes, at -12 °C for 5 minutes to 10 minutes, and at -42 °C for 0.5 minutes to 1 minute, and then, a second freezing step in which the freezing was performed at -25 °C for 1 minute to 3 minutes and at -15 °C for 1 minute to 3 minutes, and then, a third freezing step in which the freezing was performed at -42 °C for 20 to 40 minutes and at -120 °C for 20 minutes to 50 minutes; or a first freezing step in which the freezing was performed in the temperature range of 4 °C to - 40 °C at 3 °C/m, and then, a second freezing step in which the freezing was performed in the temperature range of -40 °C to - 90 °C at 5 °C/m, and then, a third freezing step in which the freezing was performed in the temperature range of -90 °C to -120 °C at 5 °C/m. The frozen cells were transferred to LN2 tank and stored (-130 °C or lower).

Upon thawing, the heat block was set to be 37 °C, and then the culture medium to which 10% plasma was added was placed in a T flask. Depending on the cell concentration, the volume of the culture medium is able to be variously adjusted to be, for example, 4 ml, 6 ml, 8 ml, or 10 ml. The cryogenic vial was placed in a heat block to thaw the frozen cells. When the frozen cells were half-thawed, they were transferred to a T flask containing a culture medium. Subsequently, the cells were placed in a 37°C, 5% CO₂ incubator and cultured for one day. The cultured cells were collected in a tube, and then, Ca/Mg free DPBS was added thereto, followed by centrifuging at 1,500 rpm for 5 minutes, and the supernatant was removed therefrom. The cells separated by centrifugation were suspended in a small amount of culture medium, and then, the number of cells was counted.

### 2. Culture of natural killer cells

### 2.1. Preparation of culture flasks on which fibronectin and gamma globulin were coated

0.01 ml fibronectin (#FC-010, Millipore) and 0.121 ml gamma globulin (#020A1004, Green Cross) solution were placed in a 15 ml tube, and then 9.859 ml of Ca/Mg glass DPBS was added. The prepared coating solution was put into a T75 flask (# 156499, Nunc) using a pipette and reacted at 2 °C to 8 °C for 16 hours or more. Before the cell culture, the remaining coating solution was removed after washing with Ca/Mg free DPBS.

### 2.2. Primary culture of natural killer cells

The cell suspension of CD3-CD56+ natural killer cells frozen and thawed in Example 1.2 was taken and inoculated into the coated flask prepared in Example 2.1, and without using feeder cells, IL-2, anti-NKp46 antibody and donor plasma were added thereto to culture the cells for 3 days to 5 days at 37 °C and 5% CO₂ condition.

### 2.3. Secondary culture of natural killer cells

After the primary culture of Example 2.2, the T75 flask in which the cells were being cultured was taken from the incubator, and the cells were collected. Then, the remaining donor plasma or FBS were added to the obtained cells to prepare a cell suspension, which was then added to a 2L CO₂ permeable culture bag containing the culture medium. The culture medium and the cell suspension were mixed well, and then, the result was placed in a 37°C, 5% CO₂ incubator and then cultured for 7 days to 14 days to proliferate cells. After the culture was completed, the final culture medium was placed in a centrifuge tube and the cells were harvested through several centrifugations.

### Experimental example 1. Analysis of purity and proliferation ability of natural killer cells

With respect to CD3-CD56+ natural killer cells 2 × 10⁸ (cells) derived from two donors cultured by the culture method according to the example described above, the number of cells was confirmed at 8, 14, and 21 days after culture, and the cells were stained using CD3 and CD56 antibodies to perform FACS assay using a known method to obtain the ratio of CD3-CD56+ natural killer cell group.

As a result, the ratio of CD3-CD56+ natural killer cell group was significantly high from the beginning of culture (culture day 0: 96.0%), and the ratio of CD3-CD56+ natural killer cell group continued to increase during the culture period (CD3-CD56+ average natural killer cell ratio of Donor 1 and Donor 2; culture day 8: 96.4%, and culture day 14: 98.3%).

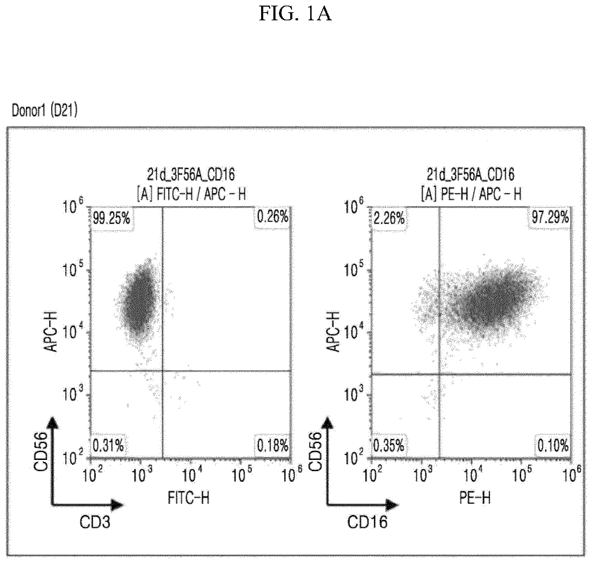

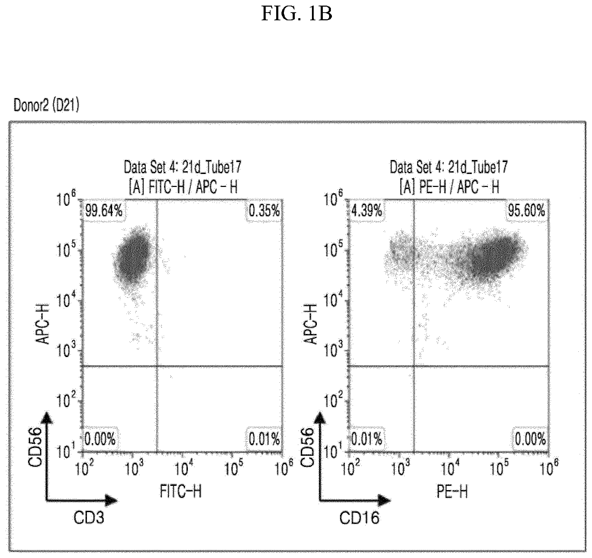

In particular, on the 21st day after culture, CD3-CD56+ NK cells accounted for 99.2% and 99.6%, confirming that natural killer cells were present at a high purity compared to before culture (FIG. 1).

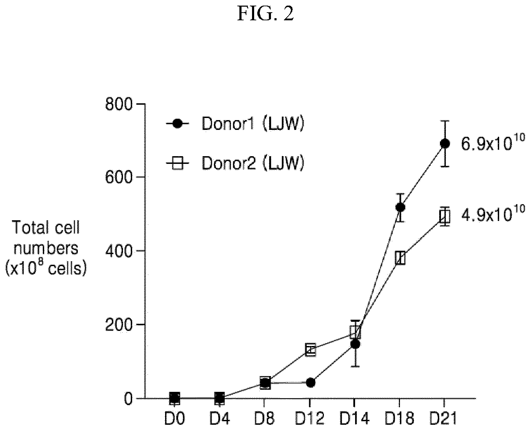

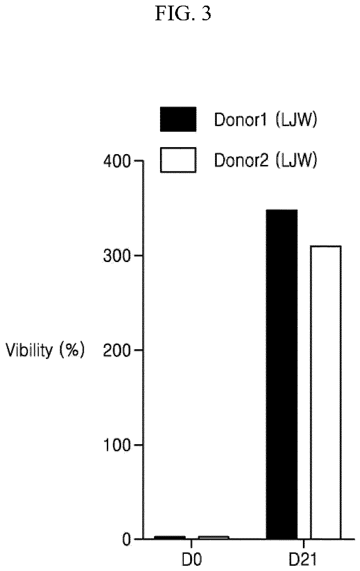

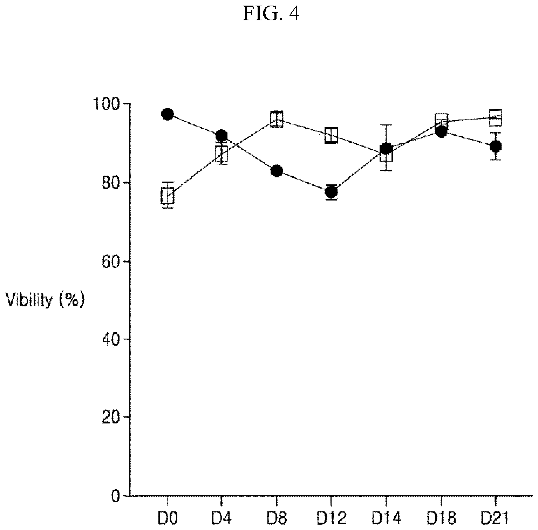

In addition, CD3-CD56+ natural killer cells derived from two donors proliferated an average of 113±27 times on day 14 after culture, and on day 21 after culture, natural killer cells proliferated to 6.9 × 10¹⁰ (cells) and 4.9 × 10¹⁰ (cells). Accordingly, it was confirmed that the natural killer cells were significantly increased with an average growth factor of 300 to 350 times (FIGS. 2 and 3). In addition, it was confirmed that all exhibited a high viability of 90% or more on average during the culture period for 21 days (FIG. 4).

Through these results, it was found that the natural killer cell according to one aspect was significantly increased in the number of cells even when cultured in vitro for a long period of time, and was stably cultured to such an extent that the viability reached 90%. In addition, since the culturing is performed by the leukapheresis and CD3-CD56+ natural killer cell selection, compared to a density gradient separation method, high-purity natural killer cells could be obtained containing few other impurities.

### Experimental example 2. Analysis of cancer cell killing ability of natural killer cells

In order to evaluate the cancer-cell killing ability of the natural killer cells cultured by the culture method according to the example, the natural killer cells on the 21st day of culture were used, and the blood cancer cell line K562, which has high sensitivity to natural killer cells, was used as a target cell.

First, target cancer cells (K562) were collected, and centrifuged at 1500 rpm for 5 minutes, and the supernatant was removed therefrom. Then, the cells were diluted with DPBS and washed, and the washed cell pellet was suspended in a culture medium, which is a Phenol Red-free RPMI medium supplemented with 10% FBS. 1×10⁵ cells per condition were prepared, and then left and stained using the concentration of 5 µM CFSE (Life technologies) in an incubator under 5% CO₂ conditions for 10 minutes. After washing twice with DPBS, the mixture was diluted with culture medium, which is a Phenol Red-free RPMI medium supplemented with 10% FBS. Activated natural killer cells were prepared according to the E:T ratio (1:1, 1.25:1, 2.5:1, 5:1, 10:1, 20:1) with the target cells, and were dispensed and mixed together with the target cells in a 24-well plate. The reaction was performed for 4 hours, and 20 minutes before the end of the reaction, 7AAD (7-Aminoactinomycin D) was treated thereon. After the reaction was completed, the cells were collected using a 5 ml FACS tube, and the killing ability of the cells was analyzed using a flow cytometer.

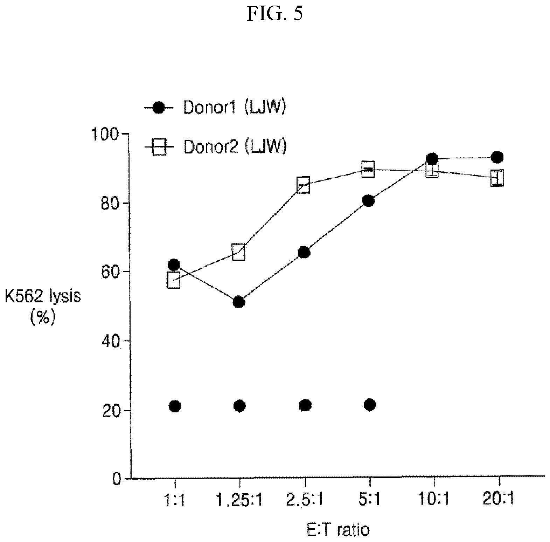

As a result, it was found that natural killer cells cultured by the culture method according to one aspect exhibit a significantly increased cell killing ability of 90% or more at the E:T (effective cell: target cells) ratio of 10:1 and 20:1, and especially even at a ratio of the E:T ratio of 1: 1 to 1.25: 1, the cells showed a significant killing ability of 80% or more (FIG. 5).

Considering these results, the natural killer cells cultured by the culture method according to one aspect is high-purity natural killer cells whose purity has increased to a level exceeding 99% through the culture process according to the examples, and shows a high proliferation efficiency and high cancer cell killing ability.

### Experimental example 3. Analysis of anticancer-related gene expression in natural killer cells

### 3.1. Analysis of mRNA expression patterns of anticancer related genes in natural killer cells

The mRNA expression patterns of anticancer-related genes in natural killer cells immediately after selection (D0) and after 14 days of culture (D14) of natural killer cells derived from three donors cultured by the culture method according to these examples, were analyzed.

Specifically, RNA was extracted from cells before and after culture by a trizol separation method, and total RNA sequencing was performed to confirm mRNA expression patterns (fold change, D14 natural killer cells/D0 natural killer cells). The expression pattern was expressed as the average value of three donors, and it was considered to be statistically significant when the corrected p-value, q-value, was 0.001 or more. The results are shown in FIG. 6 and Table 1.

**[Table 1]**

| **Function** | | **Ggene** | **Increase/D ecrease** | **Fold change** | **q-value** |
|---|---|---|---|---|---|
| NKR | Activating Receptor | NKp44 (NCR2) | Increase | 1064 times | 0.000325856 |
| | | NKp30 (NCR3) | Increase | 5.2 times | 0.000163883 |
| | Inhibitory Receptor /molecule | KIR3DL2 | Decrease | 1/2 | 0.035349 |
| | | SIGLEC9 | Decrease | 1/20 | 0.012559253 |
| | Activating molecules | DAP10 (HCST) | Increase | 1.7 times | 0.023375131 |
| | | IL 2RA | Increase | 5.6 times | 0.045398917 |
| Cytotoxicity | Necrosis/apopt osis | FAS-L (FASLG) | Increase | 6.1 times | 0.009639623 |
| | | Granzyme A (GZMA) | Increase | 6.7 times | 0.000217593 |
| | | Granzyme K (GZMK) | Increase | 10.7 times | 0.002030825 |
| | | Perforin (PRF1) | Increase | 2.1 times | 0.002984078 |
| | | TRAIL/Apo-2L (TNFSF10) | Increase | 24.5 times | 0.000134563 |
| Cytokine | Pro-inflammation (anti-tumor) | GM-CSF (CSF2) | Increase | 4.1 times | 0.038705 |
| | Anti-inflammation (immune suppression) | IL-8 (CXCL8) | Decrease | 1/100 times | 0.004886948 |
| | | IL 10 | Decrease | 1/1000 times | 0.001149443 |
| chemokine s | Recruit other effector cells | CCL1 | Increase | 318 times | 0.007551016 |
| | | CCR5 | Increase | 49.9 times | 0.000202001 |
| Apoptosis | Induction | Noxa (PMAIP1) | Decrease | 3/100 times | 0.002485492 |
| | Suppression | BCL-2 | Increase | 10.5 times | 8.51E-05 |

As a result, it was confirmed that activation receptors NKG2D, NKp30, NKp44, and NKp46 were increased, and from among them, the increase in NKp44 (1064 times) and NKp30 (5.2 times) was distinct. In addition, in the case of CD16, which mediates antibody-dependent cell-mediated cutotoxicity (ADCC) of target cells, it was confirmed that a high expression level was maintained even during culture and freezing.

NKp44 of natural killer cells activates natural killer cells by binding to cancer cell ligands mixed-lineage leukemia protein-5 (MLL5), heparin sulfate proteoglycans (HSPG), and platelet-derived growth factor (PDGF). NKp30 activates natural killer cells by binding to cancer cell ligands BAG6 and B6-H6. Accordingly, natural killer cells according to one aspect are expected to be more effective in carcinomas which highly express NKp44 ligands, such as MML5, HSPG, BAG6, and B6-H6, and NKp30 ligands. Tumors in which PDGF is increased, include glioma, gastro-intestinal stromal tumor, chronic myelomonocytic leukemia, and breast cancer. In particular, PDGFα is reported to be highly correlated with lymph node metastasis of breast cancer, high histologic grade, ER/PR negativity, and triple-negative breast subtype (Breast Cancer Res Treat. 2018; 169(2): 231-241). Therefore, natural killer cells according to one aspect are expected to be effective against these tumors.

Meanwhile, among chemokines, CCL1 (318 times) and among chemokine receptors, CCR5 (49.9 times) were increased remarkedly. Immune cells of which expression is increased, may recruit other effector cells such as monocytes, B-cells, and dendritic cells and are more advantageous in killing tumor cells. In particular, when the expression of CCR5 is increased, the cells may have affinity for CCL5+ tumor cells, thereby being more advantageous for killing these tumors. CCL5 is highly expressed in metastatic/advanced breast cancer, cervical cancer, gastric cancer, colon cancer, prostate cancer, ovarian cancer (Mediators of Inflammation Volume 2014, Article ID 292376), etc. Accordingly, natural killer cells according to an aspect, having increased CCR5, may have a higher affinity for tumors.

As shown in Table 1, the expression of NKp44 and NKp30, which are factors related to activation of natural killer cells, CCL1, which is a chemokine that mobilizes other effector cells, and CCR5, which is a chemokine receptor, were increased from 5.2 times to 1064 times, and the expression of the inactivation receptors KIR3DL2 and Siglec9, which inactivate natural killer cells when bound to ligand, were significantly reduced by 0.5 times and 0.05 times, respectively. When KIR3DL2 binds to cancer cell ligands, such as HLAA3, A11, and B27 homodimer, the production of INF-γ and cancer cell killing ability of natural killer cells are reduced. When Siglec9 binds to sialic acid in cancer cells, ITIM phosphorylation is induced in natural killer cells, and SHP-1 and SHP-2 are gathered to inhibit cell killing ability. Therefore, natural killer cells according to one aspect in which the expression of these inactive receptors is significantly reduced, are expected to further increase cancer cell death.

In addition, the cell killing ability of natural killer cells is comprehensively achieved through two mechanisms. One is the secretion of granular substances such as Granzyme and Perforin in the cytoplasm to kill cancer cells, and the other is the apoptosis of cancer cells through binding to TRAIL, and/or FASL, which are the ligands that recognizes Fas or tumor necrosis factor receptor (TNFR), which are the death receptor of cancer cells. In the case of natural killer cells according to one aspect, it was confirmed the expression of Ganzyme A, Granzyme K, and Perforin, which are factors related to anticancer activity (cell killing ability), was increased as small as 2.1 times and as large as 10.7 times, and the expression of FAS-L (FASLG) and TRAIL (TNFSF10), which induce the increase in apoptosis, was increased as small as 6.1 times to as large as 24.5 times.

In addition, the expression of BCL-2, which delays the apoptosis of natural killer cells, was significantly increased by 10.5 times, and the expression of Noxa, which promotes apoptosis, was significantly decreased by 0.03 times.

### 3.2. Differences in anticancer-related gene expression patterns according to CD3-CD56+ natural killer cell selection

Anticancer-related gene expression patterns were analyzed, of natural killer cells cultured after CD3-CD56+ selection using the leukapheresis technique of Example 1 and the CliniMACS Prodigy^{®} system, and natural killer cells cultured without the selection process. In the same manner as described in Experiment Example 3, RNA was extracted from cells before and after culture by a trizol separation method, and total RNA sequencing was performed to confirm mRNA expression patterns (fold change, D14 natural killer cells/D0 natural killer cells). The expression pattern was expressed as the average value of three donors, and it was considered to be statistically significant when the corrected p-value, q-value, was 0.001 or more. The results are shown in Table 2.

**[Table 2]**

| **Function** | | **Ggene** | **Increase/D ecrease** | **Fold change** | **q-value** |
|---|---|---|---|---|---|
| NKR | Activating Receptor | NKp44 (NCR2) | Increase | 7.2 times | - |
| | | NKp30 (NCR3) | Increase | 19.3 times | 0.028 |
| Cytotoxicity | Necrosis/apopt osis | FAS-L (FASLG) | Increase | 16.0 times | 0.015 |
| | | Granzyme A (GZMA) | Increase | 41.9 times | 0.01 |
| | | Granzyme K (GZMK) | Increase | 5.4 times | 0.01 |
| | | Perforin (PRF1) | Increase | 9.7 times | 0.09 |
| | | TRAIL/Apo-2L (TNFSF10) | Increase | NA(0.11) | - |
| Cytokine | Anti-inflammation (immune suppression) | IL-8 (CXCL8) | Decrease | NA(0.11) | - |
| | | IL 10 | Decrease | 16.75 times | 0.015 |
| chemokine s | Recruit other effector cells | CCL1 | Increase | 1.1 times | 0.5 |
| | | CCR5 | Increase | 1.7 times | 0.09 |
| Apoptosis | Induction | Noxa (PMAIP1) | Decrease | 7.2 times | - |
| | Suppression | BCL-2 | Increase | 19.3 times | 0.028 |

As a result, it was confirmed that the natural killer cells cultured through CD3-CD56+ selection using the leukapheresis technique and the CliniMACS Prodigy^{®} system of Example 1 had the following characteristics compared to those without the same.
a) From among the activation receptors, the expression of NKp44 was significantly increased (1064 times vs. 7.2 times).
b) In the case of cell killing ability, the expression of granzyme K (10.7 times) and TNFSF10 (24.5 times) was increased significantly. On the other hand, when CD3-CD56+ selection was not performed, the expression of granzyme A was increased significantly (41.9 times).
c) The expression of IL-8 (1/100 times) and IL-10 (1/1000 times) was significantly decreased.
d) The expression of CCR5, which is a chemokine receptor that recruits other effector cells, was markedly increased (49.9 times vs. 16.75 times).
e) The increase in the expression of BCL-2, which delays self-death of natural killer cells (10.5 times) was clearly shown, and the expression of Noxa,
which promotes self-death was clearly shown (0.03 times).

### 3.3. Comparison of expression patterns of activated receptors between natural killer cells and human NK cell lines according to one aspect

Expression patterns of activated receptors of natural killer cells (pNK) cultured by culture methods according to the aspect were compared with representative human NK cell lines, NK92mi and NK92. Human NK cell lines, NK92mi and NK92, were purchased from American Type Culture Collection (ATCC), and the expression levels of NKG2D, NKp46, NKp44, NKp30, DNAM-1, and CD16, which are representative activation receptors of natural killer cells, were analyzed using flow cytometer. The results are shown in Table 3 below.

**[Table 3]**

| **Target** | | **pNK (n=3)** | **NK92mi (n=3)** | **NK92 (n=3)** |
|---|---|---|---|---|
| | | **Mean (%) ± SD** | **Mean (%) ± SD** | **Mean (%) ± SD** |
| Activation receptor | CD314 (NKG2D) | 80.13 | 79.60 | 50.17 |
| | CD335 (NKp46) | 54.77 | 4.17 | 1.38 |
| | CD336 (NKp44) | 95.43 | 27.27 | 10.38 |
| | CD337 (NKp30) | 93.20 | 83.30 | 87.13 |
| | CD226 (DNAM-1) | 99.27 | 3.58 | 1.41 |
| | CD16 | 90.60 | 1.80 | 23.60 |

As a result, it was confirmed that, in natural killer cells according to an aspect, NKG2D (80.13%), NKp44 (95.43%), NKp30 (93.20%), DNAM-1 (99.27%), and CD16 (90.60%), which are representative activation receptors, were all expressed at a high ratio of 80% to 99%, and the expression of NKp46 was as high as about 55%. On the other hand, in the case of human NK cell lines, NK92mi and NK92, it was confirmed that the expression rate of the activation receptor was significantly lower than that of natural killer cells according to one aspect.

### Experimental Example 4. Cytotoxicity test of natural killer cell

### 4.1. Cytotoxicity test using flow cytometry

The cytotoxicity of natural killer cells (pNK) cultured by the culture method according to one aspect was confirmed through flow cytometry. The 7AAD-CFSE method, which is useful for cytotoxicity testing using floating cells such as K562 cells without using radioactive isotopes, was performed. As target cells, K562, which is a chronic myelogenous leukemia cell line, cisplatin-sensitive A2780 and resistant cell A2780cis, which are ovarian cancer cell lines, and MCF7, SKBR3, and T47D, which are breast cancer cell lines, were cultured the day before the test, and then used. As effector cells, NK92mi, which is a representative human NK cell line, was used as a control, along with natural killer cells (pNK) according to one aspect.

As a result, compared to representative human NK cell lines, natural killer cells cultured by the culture method according to one aspect showed a substantial increase in the cancer cell removal ability according to the E:T ratio not only in K562, which is a blood cancer cell line, but also in various solid cancers such as ovarian cancer and breast cancer (FIG. 7).

These results show that the natural killer cells cultured by the culture method according to one aspect exhibit a substantial increase in the expression of anticancer-related genes and the expression of activation receptors, leading to an increase in the cancer cell killing effect.

### 4.2. Evaluation of anticancer activity in ovarian cancer mouse model

The efficacy was evaluated by confirming the growth inhibitory effect of natural killer cells (pNK) cultured by the culture method according to one aspect as an immuno-anticancer agent. An A2780cis xenograft mouse model was prepared by subcutaneously injecting the mouse ovarian cancer cell line A2780cis into the fat pad of 7-week-old NSG mice in an amount of 2 × 10⁶ (cells). After ovarian cancer cell lines were injected, mice with tumors were randomly grouped on day 3: a group treated with intraperitoneal injection of cisplatin at 1 mg/kg, once/week; a group treated with intravenous injected of natural killer cells according to one aspect in the number of 1 × 10⁷ (cells), 2 times/week; and a control group treated with the injection of PBS (FIG. 8). After injection of the ovarian cancer cell line, tumor size and body weight were measured daily.

As a result, it was confirmed that when the natural killer cells cultured by the culture method according to one aspect were administered alone, 80% or more of a remarkable cancer cell growth inhibitory effect was obtained compared to the control group. On the other hand, when cisplatin, an existing anticancer drug, was injected, about 55% of the cancer cell growth inhibition effect was obtained (FIG. 9, A). On the other hand, no significant change in body weight was observed in both groups respectively treated with the natural killer cells according to one aspect and cisplatin-administered group (FIG. 9, B). The weight of tumors extracted after sacrifice of mice was reduced by 70% or more on average compared to the control group and reduced by 50% or more on average compared to the cisplatin-treated group (FIG. 9, C)

Through the results, it was confirmed that the anticancer activity and the activity of the cell itself were increased in the natural killer cell cultured by the culture method according to one aspect, and the self-death of the natural killer cell was suppressed to increase the viability in vivo. It was confirmed that these characteristics appeared distinctly when cultured through leukapheresis and CD3-CD56+ selection. In addition, as can be seen from the results of the animal model, it was confirmed that the natural killer cell cultured by the culture method according to one aspect showed an excellent anticancer effect of significantly reducing the size of the tumor.

Therefore, the natural killer cells according to one aspect is cultured after being selected from a healthy donor in large quantities. Compared to culture using conventional peripheral blood mononuclear cells, high-purity natural killer cell culture may be obtained with high safety and increased cancer cell killing effect. Accordingly, the natural killer cells can be used as cell therapy for efficient cancer treatment. In addition, since feeder cells are not used during culture, safety is ensured clinically. Accordingly, the natural killer cells are expected to be useful as a new allogeneic immune cell therapy with enhanced function.

## Claims

1. A method of culturing natural killer cells, the method comprising:
1) selecting mononuclear cells from human peripheral blood through leukapheresis;
2) obtaining CD3-CD56+ natural killer cells from the selected mononuclear cells; and
3) treating the obtained CD3-CD56+ natural killer cells with IL-2, anti-NKp46 antibody, and plasma, followed by culturing in a feeder cell-free culture medium.

2. The method of claim 1, wherein natural killer cells cultured using the method have one or more characteristics selected from the following (a) to (e) compared to natural killer cells before culture:
(a) an increase in the expression of one or more selected from the group consisting of NKG2D, NKp30, NKp44, and NKp46;
(b) a decrease in the expression of one or more selected from the group consisting of KIR3DL2 and Siglec9;
(c) an increase in the expression of one or more selected from the group consisting of FASL and TRAIL (TNFSF10);
(d) an increase in the expression of one or more selected from the group consisting of Granzyme A, Granzyme K, and Perforin; and
(e) an increase in the expression of one or more selected from the group consisting of CCL1 and CCR5.

3. The method of claim 2, wherein natural killer cells cultured using the method further have one or more characteristics selected from the following (f) to (j) compared to natural killer cells before culture:
(f) an increase in the expression of one or more selected from the group consisting of DAP10 (HCST), and IL-2RA;
(g) an increase in the expression of GM-CSF (CSF2);
(h) a decrease in the expression of one or more selected from the group consisting of IL-8 (CXCL8), and IL-10;
(i) a decrease in the expression of Noxa (PMAIP1); and
(j) an increase in the expression of BCL-2.

4. The method according to claim 1, wherein the natural killer cells cultured according to the method have increased purity, increased cell proliferation fold, and increased cancer cell killing ability, compared to natural killer cells before culture.

5. The method of claim 1, wherein step 1) is performed through leukapheresis.

6. The method of claim 1, wherein step 2) is performed using a closed system.

7. The method of claim 1, wherein the CD3-CD56+ natural killer cells in step 3) are cryopreserved and then thawed.

8. The method of claim 1, wherein the plasma of step 3) is separated from human peripheral blood.

9. The method of claim 1, wherein the culturing in step 3) is performed in the presence of gamma globulin and fibronectin.

10. The method of claim 1, wherein the culturing in step 3) is to culture natural killer cells by culturing peripheral blood mononuclear cells.

11. A composition for culturing natural killer cells, comprising IL-2, anti-NKp46 antibody, and plasma, wherein the composition does not include a feeder cell.

12. The composition of claim 11, wherein the natural killer cells cultured by the composition have one or more characteristics selected from the following (a) to (e) compared to natural killer cells before culture:
(a) an increase in the expression of one or more selected from the group consisting of NKG2D, NKp30, NKp44, and NKp46;
(b) a decrease in the expression of one or more selected from the group consisting of KIR3DL2 and Siglec9;
(c) an increase in the expression of one or more selected from the group consisting of FASL and TRAIL (TNFSF10);
(d) an increase in the expression of one or more selected from the group consisting of Granzyme A, Granzyme K, and Perforin; and
(e) an increase in the expression of one or more selected from the group consisting of CCL1 and CCR5.

13. The composition of claim 12, wherein the natural killer cells cultured according to the method further have one or more characteristics selected from the following (f) to (j) compared to natural killer cells before culture:
(f) an increase in the expression of one or more selected from the group consisting of DAP10 (HCST), and IL-2RA;
(g) an increase in the expression of GM-CSF (CSF2);
(h) a decrease in the expression of one or more selected from the group consisting of IL-8 (CXCL8), and IL-10;
(i) a decrease in the expression of Noxa (PMAIP1); and
(j) an increase in the expression of BCL-2.

14. The composition of claim 11, wherein the plasma is separated from human peripheral blood.

15. The composition of claim 11, wherein the composition further comprises gamma globulin and fibronectin.

16. A natural killer cell cultured by the method of claim 1 or a cell population thereof.

17. A pharmaceutical composition for preventing or treating cancer comprising the natural killer cell or the cell population thereof of claim 16 as an active ingredient.

18. The pharmaceutical composition of claim 17, wherein the cancer is at least one selected from the group consisting of glioma, gastrointestinal stromal tumor, leukemia, breast cancer, uterine cancer, cervical cancer, gastric cancer, colon cancer, prostate cancer, ovarian cancer, lung cancer, laryngeal cancer, rectal cancer, liver cancer, gallbladder cancer, pancreatic cancer, prostate cancer, kidney cancer, skin cancer, bone cancer, muscle cancer, fat cancer, fibrocyte cancer, hematological cancer, lymphoma, and multiple myeloma.

19. A method of preventing or treating cancer, comprising administering the pharmaceutical composition of claim 17 to a subject.

20. Use of the pharmaceutical composition of claim 17 for the manufacture of a medicament for preventing or treating cancer.
